# FASCICULE DE BREVET EUROPEEN

(11) **EP 1 036 013 B1**
(45) Date de publication et mention de la délivrance du brevet: **28.07.2004**
(21) Numéro de dépôt: 98954510.8
(22) Date de dépôt: 04.11.1998
(51) Int. Cl.: B65D 47/18

(54) **DISPOSITIF DE CONDITIONNEMENT POUR LIQUIDE A DISTRIBUER GOUTTE A GOUTTE**
VORRICHTUNG ZUM AUFBEWAHREN VON FLÜSSIGKEIT MIT TROPFENSPENDER
DEVICE FOR PACKAGING A LIQUID TO BE DISPENSED DROP BY DROP

(30) Priorité: 04.11.1997 FR 9713863
(43) Date de publication de la demande: 20.09.2000
(73) Titulaire: Laboratoires THEA, 63100 Clermont-Ferrand (FR)
(72) Inventeur: CHIBRET, Henri, F-63350 Joze (FR); FAURIE, Michel, F-63960 Veyre-Monton (FR); LUYCKX, Jacques, F-63112 Ceyrat (FR); DEFEMME, Alain, F-63400 Chamalières (FR); COQUEL, Thierry, F-63400 Chamalières (FR)
(74) Mandataire: Thibon-Littaye, Annick
(86) Numéro de dépôt international: PCT/FR1998/002353
(87) Numéro de publication internationale: WO 1999/023006

(56) Documents cités:
- EP-A- 0 425 264
- WO-A-90/05110
- DE-A- 19 618 750
- US-A- 3 669 323
- US-A- 4 634 023
- US-A- 5 105 993

## Description

La présente invention concerne l'industrie du conditionnement des liquides, plus particulièrement quand il s'agit de la conception et de la fabrication de récipients à obturateur distributeur incorporé qui servent au conditionnement de liquides à distribuer goutte à goutte.

Les dispositifs de ce genre connaissent de nombreux domaines d'application, et pas seulement dans le monde des produits pharmaceutiques ou cosmétiques, mais aussi, par exemple pour des lubrifiants ou des solutions utilisées dans l'industrie agro-alimentaire. Par leur emploi, on cherche principalement à conserver longtemps des produits liquides que l'on n'utilise que progressivement par petites doses, en expulsant juste quelques gouttes à chaque fois.

Bien souvent, le produit conservé dans le flacon doit en plus être protégé de toute contamination. Tel est le cas par exemple des solutions ophtalmiques, que l'on doit maintenir isolées de l'air ambiant et des bactéries qu'il contient. En pratique, la préservation de telles solutions passe par deux voies, que l'on peut d'ailleurs utiliser simultanément. L'une consiste à disposer un filtre anti-bactérien sur le canal d'expulsion des gouttes, l'autre à incorporer dans le liquide un agent conservateur qu'il convient alors de retenir pour en épurer le produit expulsé.

En art antérieur à la présente invention, on connaît des dispositifs de conditionnement de liquides à distribuer goutte à goutte, qui comprennent un flacon à volume intérieur variable par déformation manuelle de ses parois, et un embout distributeur renfermant un élément de purification interposé sur le trajet suivi par le liquide lors de son expulsion hors du flacon sous l'effet de la pression exercée par diminution du volume de ce dernier.

De tels dispositifs de conditionnement sont décrits, par exemple, dans le brevet français 2 638 428 de la demanderesse. On y voit que dans l'embout distributeur, le canal d'expulsion du liquide est précédé d'un sas limité par un élément d'épuration amont, apte à retenir le conservateur d'une solution ophtalmique constituant le liquide à distribuer, et par un élément de filtration aval, constitué par exemple par une membrane anti-bactérienne.

Toutefois, les dispositifs connus présentent encore bien des inconvénients, auxquels la présente invention apporte solution. Quand en particulier, ils sont utilisés pour le traitement de maladies de l'oeil, il convient d'instiller le liquide goutte à goutte dans l'oeil du patient, et cette opération est d'autant plus délicate qu'elle est couramment effectuée par le patient lui-même.

D'une manière plus générale, ces dispositifs n'apportent pas la précision souhaitée dans le dosage correct du produit. D'une part, il est difficile d'exercer une pression d'expulsion répétitive lente et régulière comme il le faudrait, et si par exemple, quand on presse sur la paroi externe du flacon, on agit trop rapidement, on ne parvient pas à former les gouttes et on expulse trop de produit d'un seul coup. D'autre part, la pression à exercer par l'utilisateur varie au cours de l'emploi du dispositif. Elle augmente au fur et à mesure que le produit est consommé, et en fin de vie utile il reste toujours un volume mort de liquide non utilisable dans le flacon.

L'invention vise donc à permettre d'obtenir un débit de liquide faible et régulier comme il est souhaitable pour assurer la formation des gouttes, en échappant à l'influence des conditions de manipulation des parois déformables du flacon, afin que le distributeur puisse fonctionner véritablement en compte-gouttes. Elle vise aussi à permettre une utilisation plus complète du produit introduit initialement dans le flacon.

Par ses différentes caractéristiques, telles qu'elles seront définies et décrites ci-après et telles qu'elles peuvent être avantageusement appliquées industriellement, l'invention n'assure pas seulement la maîtrise de la formation et de l'éjection des gouttes individuelles. Elle permet en outre d'améliorer la sécurité de fonctionnement du dispositif de conditionnement dans ses autres fonctions, telles que l'état de pureté du produit, de faciliter sa fabrication à l'échelle industrielle et de diminuer les coûts.

Pour parvenir à ses différents objectifs, l'invention propose un dispositif de conditionnement pour liquide à distribuer goutte à goutte tel que defini dans la revendication 1, comportant notamment un flacon à paroi flexible déformable manuellement en diminution progressive de son volume interne vers un col rigide dans lequel se monte une tête de distribution terminée par un canal d'expulsion du liquide hors du flacon, caractérisé en ce qu'il comporte un tampon microporeux s'insérant dans le corps de la tête de distribution en travers de la section de passage du liquide entre le flacon et le canal d'expulsion, en amont d'une chambre de répartition du liquide ménagée entre une face aval du tampon et le canal d'expulsion.

Ce tampon a pour effet de créer une perte de charge régulatrice du flux de liquide poussé à travers lui par la pression engendrée dans le flacon lors de sa réduction de volume. La dose qui le traverse à chaque action de l'utilisateur en compression du flacon se regroupe dans le volume tampon de la chambre de répartition avant de sortir par le canal d'expulsion sous forme de gouttes bien distinctes. A ce sujet, il semble que le dispositif suivant l'invention soit capable de fonctionner particulièrement bien lorsque la chambre de répartition est de volume suffisant pour recevoir une dose d'une à trois gouttes de liquide.

Un tampon microporeux donnant satisfaction suivant l'invention est avantageusement réalisé en une matière inerte vis-à-vis du liquide contenu dans le flacon. Des matières appropriées seront en particulier les feutres ou les mousses à haute porosité en pores ouverts telles qu'on sait les obtenir à partir de résines diverses de polymères organiques. Dans les principales applications de l'invention, il est avantageux de réaliser le tampon microporeux sous la forme d'une pastille de feutre de résines de polyesters ou polyesters modifiés, telles en particulier que les résines de polyéthylène à basse densité ou les résines de polyéthersulfone.

Les résines de ce type ou équivalentes, qui sont bien entendues connues en elles-mêmes, ont l'intérêt, dans le cadre de l'invention, de se prêter à fournir un tampon cylindrique, dans les diamètres de 0,5 à 3 cm et des longueurs comprises entre 0,2 et 1 cm, qui présente une souplesse suffisante pour s'adapter en force de manière étanche dans le corps, avantageusement cylindrique, de la tête de distribution, et qui, dans le sens longitudinal, offre au passage du liquide des microcanaux d'un diamètre moyen de pores pouvant être choisi entre 0,3 et 10 microns.

Les gammes de dimensions ci-dessus conviennent bien pour assurer l'effet recherché dans le cas notamment de solutions aqueuses de médicaments, telles que les solutions de traitement de la cornée ou toute autre solution destinée à être administrée en gouttes dans l'oeil.

Dans un sas tel que mentionné ci-dessus à propos de l'art antérieur illustré par le brevet français de la demanderesse publié sous le numéro 2 638 428, le tampon du dispositif de l'invention est configuré et disposé pour occuper ledit sas sur toute sa section transversale, et ce de préférence sur un à deux tiers de sa hauteur longitudinale, le complément de cette hauteur étant réservé à la chambre de répartition.

Combiner ledit tampon avec un élément de filtration aval du type d'une membrane antibactérienne est également avantageux, afin d'assurer la protection du liquide contenu dans le flacon en évitant sa contamination à partir de l'air ambiant. Une membrane particulièrement bien adaptée présente couramment une épaisseur de quelques dixièmes de millimètres et une dimension moyenne de maille comprise entre 0,2 et 0,8 microns. Imprégnée de solution, elle empêche la pénétration de l'air ambiant dans le flacon en remplacement du liquide expulsé. Il est donc clair que pour lui-même, le tampon préconisé par l'invention en est totalement différent, et dans sa structure et dans sa fonction.

En liaison avec la présence d'un élément aval de filtration ou purification avantageusement constituée par une membrane anti-bactérienne, l'invention présente en caractéristique secondaire le fait de disposer cet élément plaqué contre un embout raccordé au corps de la tête de distribution dans lequel est ménagé le canal d'expulsion. En pratique, il faut comprendre ici que la membrane est soutenue plane entre deux plateaux qui sont néanmoins ajourés, de manière à éviter que entre deux étapes de distribution, il reste du liquide mouillant la membrane, ce qui nuirait à son fonctionnement.

A titre d'exemple préféré, un tel plateau peut être réalisé dans la matière de la tête de distribution par des ailettes en étoile formant écarteur entre la face aval du tampon microporeux et la membrane anti-bactérienne. En coopération avec elles, l'embout présentera avantageusement des rainures en secteurs annulaires concentriques autour d'un orifice central du canal d'expulsion.

Par ailleurs, la tête de distribution, constituant insert ou nacelle dans le col du flacon, peut porter en plus un autre élément de purification, situé en amont du tampon microporeux, notamment un élément en soi classique destiné à débarrasser le liquide expulsé d'un agent conservateur, qui se trouve alors retenu dans le flacon, comme il est souhaitable par exemple dans le cas des solutions ophtalmiques. Conformément à d'autres modes de réalisation du dispositif de l'invention, le même tampon peut combiner plusieurs des fonctions ci-dessus. Ceci sera le cas notamment si le tampon comporte une couche de revêtement ou de traitement appropriée sur sa face amont.

Dans tous les cas, la régulation qu'assure le tampon du dispositif de l'invention sur le flux de liquide poussé à travers lui, en empêchant les surpressions exagérées de se manifester au delà du flacon lui-même, permet d'imposer la différence de pression la mieux adaptée à la formation des gouttes entre l'intérieur du flacon et l'extérieur. Mais aussi, il semble que la répartition du liquide passant dans les microcanaux de manière uniforme sur toute la section transversale de son trajet avant de se regrouper dans la chambre de répartition en sortie du tampon contribue fortement à l'effet bénéfique apporté par l'invention.

D'autres caractéristiques de l'invention sont directement liées aux précédentes dans la mesure où les conditions améliorées de formation des gouttes permettent de mieux adapter la construction de l'ensemble du dispositif aux exigences de la pratique sous des dimensions et des capacités variées et de faciliter sa fabrication à moindre coût.

Conformément à l'un des modes de réalisation du dispositif de l'invention, la paroi du flacon est d'encombrement général cylindrique en forme de soufflet déformable longitudinalement terminé par un fond transversal rigide. Elle est avantageusement réalisée d'une seule pièce de matière moulable avec le col rigide de montage de la tête de distribution et avec une bague extérieure de renforcement venant en débordement radial de l'encombrement général du soufflet de manière à permettre sa préhension par une machine d'assemblage automatique sans toucher à la paroi plus fragile dudit soufflet.

On prévoit avantageusement qu'un tel flacon en soufflet soit monté par son col à l'intérieur d'une coque extérieure de protection l'enveloppant sur une majeure partie de sa longueur. La bague extérieure ci-dessus est alors fort utile pour l'opération d'un procédé d'assemblage consistant à pousser le flacon en force dans cette coque en le manipulant par un tube en butée sur la bague.

Quand il est souhaitable que la paroi en soufflet puisse être comprimée sur la quasi-totalité de sa longueur, une solution bien adaptée consiste à donner à la coque de protection du soufflet une longueur au moins égale à celle du soufflet non comprimé tout en la munissant d'une fente d'accès à partir de son fond ouvert.

Toutefois, l'invention permet de préférer à cette solution celle suivant laquelle ladite coque est de pourtour cylindrique continu et associée à une cape de fermeture s'adaptant sur elle dans son prolongement, qui est amovible pour donner accès au fond du flacon. Ceci a non seulement l'avantage de mieux protéger le flacon souple, mais aussi celui de mieux convenir à une fabrication automatisée avec pose d'étiquettes collées autour de la coque cylindrique.

L'invention sera maintenant plus complètement décrite dans le cadre de ses caractéristiques préférées et de leurs avantages, en faisant référence aux figures des dessins annexés qui les illustrent et dans lesquelles :
- la figure 1 représente un dispositif de conditionnement de liquides aqueux conforme à la présente invention dont elle montre en coupe la partie supérieure, au niveau de la tête de distribution ;
- la figure 2a montre l'ensemble du dispositif dans sa position d'utilisation tête en bas, avec son bouchon séparé sur la figure 2b ;
- la figure 3 illustre une variante de réalisation du dispositif précédent qui en diffère par la constitution de la coque extérieure de protection du flacon en soufflet ;
- la figure 4 montre schématiquement les principaux postes d'une machine d'assemblage des différents éléments du dispositif dans une fabrication en série;
- et la figure 5 illustre l'étape de mise en place du soufflet dans sa coque que comporte le procédé d'assemblage mis en oeuvre au moyen d'une telle machine.

Le dispositif de conditionnement décrit est tel qu'il convient notamment pour des liquides aqueux à distribuer goutte à goutte, et plus particulièrement des solutions pharmaceutiques en milieu aqueux telles que les solutions dites de gouttes dans l'oeil.

Il comprend un récipient de réception du liquide constitué par un flacon 1 s'ouvrant par un col 5, une coque extérieure de protection 8 autour de ce flacon, une tête de distribution 2 dont le corps 3 forme une nacelle mobile entre deux positions dans le col 5 du flacon, et un embout 4 complétant ce corps hors du flacon qui est percé d'un canal d'expulsion 41. Un bouchon amovible 7 coiffe l'ensemble.

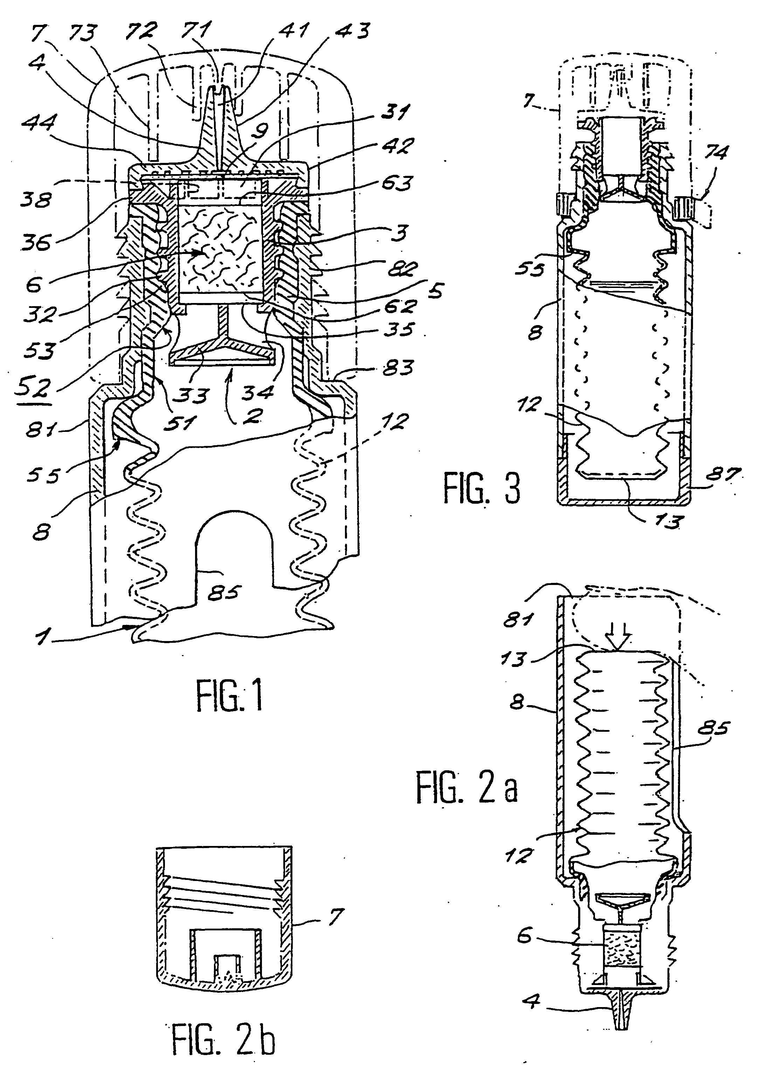

Le volume intérieur du flacon 1 est variable par action manuelle en déformation de ses parois. On peut observer des figures que, dans l'essentiel de sa partie longitudinale suivant l'axe du dispositif, verticalement dans la position représentée, sa paroi est d'encombrement général cylindrique sous la forme un soufflet 12 que l'on peut comprimer, en réduisant ainsi sa longueur. Plus précisément, et comme il ressort notamment de la figure 2, l'utilisateur actionne pour cela un fond rigide 13 du soufflet 12, qu'il pousse avec un doigt (l'index en général, quand il tient le flacon par sa tête entre le pouce et le majeur) en direction du col du flacon et de la tête de distribution associée. La réduction de volume qui en résulte s'effectue progressivement, par à-coup à chaque étape de la distribution goutte-à-goutte, dans la mesure où le dispositif est équipé de moyens empêchant l'air extérieur de rentrer dans le flacon en remplacement du liquide expulsé.

Cette dernière capacité lui est conférée, dans le mode de réalisation préféré décrit ici, par une membrane antibactérienne 9 qui est soutenue plane entre deux plateaux supports à travers toute la section de passage du liquide dans le corps 3 de la tête de distribution. Elle est en outre disposée de telle sorte qu'elle ne reste jamais mouillée par le liquide du flacon en dehors des périodes d'utilisation en distribution goutte à goutte. Ceci permet de préserver l'efficacité de la membrane dans son activité anti-bactérienne et imperméable à l'air pendant toute la durée de stockage et de vie utile du flacon. Une telle membrane, classique en elle-même, est par exemple à dimension moyenne de pores égale à 0,45 microns.

Conformément à l'invention, un tampon microporeux 6 est interposé dans le corps 3 de ladite tête de distribution, en travers de la section de passage du liquide, entre le flacon 1 et le canal d'expulsion 41. Ce tampon est constitué par un tronçon de barre cylindrique et réalisé en une matière neutre, inerte chimiquement vis-à-vis du liquide à distribuer contenu dans le flacon en tous ses constituants, donc y compris par exemple vis-à-vis d'un conservateur contenu dans une solution ophtalmique. Plus précisément, il s'agit dans l'exemple décrit d'une matière organique à base de résine de polyéthylène, qui présente une certaine souplesse élastique, telle qu'il est facile d'assurer son montage étanche dans la tête de distribution en l'insérant en force dans son corps 3, sous réserve que ce dernier présente une section interne cylindrique de même diamètre sans jeu.

On peut observer sur la figure 1 que le tampon 6 ne s'étend pas longitudinalement jusqu'à l'embouchure interne du canal d'expulsion 41. Au contraire, il est disposé en amont d'une chambre 31 qui est ménagée dans le corps cylindrique 3 entre une surface aval 63 terminant le tampon 6 et la face terminale de l'embout 4. Cette chambre présente donc une section étendue par rapport au canal d'expulsion 41, en réalité au moins aussi étendue que la section du tampon 6, et même un peu plus comme illustré par la figure 1. Elle joue le rôle d'une chambre de répartition du liquide poussé par l'utilisateur à travers le tampon, dans la mesure où, comme décrit, elle présente un volume suffisant pour recevoir le liquide ayant ainsi traversé le tampon 6, en une quantité correspondant à une dose de distribution.

Dans l'exemple particulier décrit, il est admis que le tampon 6 occupe quasiment des deux tiers de la hauteur longitudinale du corps 3, qui se situe en concordance avec le col 5 à partir de la première utilisation du dispositif, la hauteur restante étant réservé à la chambre de répartition 31 jusqu'à la base de l'embout 4, percé en son centre par l'orifice du canal d'expulsion 41. La matière microporeuse présente une dimension moyenne de pores de l'ordre de 0,5 microns.

Ainsi constitué et disposé, le tampon 6 est opératif pour créer une perte de charge régulatrice du flux de liquide poussé à travers lui par la pression engendrée dans le flacon 1 lors de chaque action en réduction de son volume. Il contribue ainsi avec la chambre 31, qui regroupe le liquide pour le répartir vers l'entrée du canal 41, avec également le mode de fonctionnement du soufflet, pour assurer une bonne distribution, véritablement en goutte à goutte, en déterminant un différentiel de pression à l'expulsion qui n'est pratiquement plus sensible à la manière dont l'utilisateur pousse sur le fond du soufflet pour le comprimer, de manière plus ou moins rapide en exerçant un effort plus ou moins grand.

S'agissant d'un flacon prévu pour un produit pharmaceutique, il convient d'isoler ce produit de toute possibilité de contamination pendant la durée de son stockage avant une première utilisation. A cet effet, le dispositif de l'invention est conçu pour permettre une fermeture étanche entre la tête de distribution et l'intérieur du soufflet 12, ce qui a en outre l'intérêt d'interdire dans le même temps toute circulation du liquide jusqu'à la membrane antibactérienne, quelle que soit la position du flacon.

Cette fermeture étanche fait intervenir la configuration de la tête de distribution en combinaison avec celle du col du flacon, plus la coopération du bouchon amovible 7 avec la coque extérieure 8, conformément à la réalisation illustrée plus particulièrement par les figures 1 et 3.

Comme le montre notamment la figure 1, la tête de distribution 2 est montée, par son corps 3, mobile dans le col rigide 5 du flacon, entre deux positions prédéterminées. Dans l'une et dans l'autre, le corps 3 reste étanche dans son montage dans le col 5, grâce à trois godrons 32 qu'il comporte en protubérances annulaires sur sa périphérie et qui se logent élastiquement dans des dégagements annulaires correspondants, ménagés dans la surface interne du col 5 sous forme de rainures se succédanr longitudinalement.

Antérieurement à la première utilisation, la tête de distribution se trouve dans la position haute dite de sécurité illustrée sur la figure 3, deux des godrons 32 se trouvant dans deux rainures du col 5, tandis que le troisième (supérieur) arrive seulement contre l'extrémité du col 5. Dans cette position, une coupelle inférieure 33 que comporte le corps 3 se trouve en contact étanche avec la surface interne de ce col, suivant une surface tronconique formant pour elle un siège 52 autour de l'axe du dispositif. La coupelle 33 ferme alors, de manière étanche, l'intérieur du flacon 1, et le liquide qu'il contient ne peut pas circuler vers le tampon 6.

Quand, au contraire, la tête de distribution est poussée vers le bas, jusqu'à la position illustrée par la figure 1, appelée ici position de disponibilité, la coupelle 33 se trouve déplacée dans une partie 51 du col rigide où le diamètre interne est plus important. Dans cette position, la communication est ouverte entre le soufflet 12 et la tête de distribution, par le biais de trois orifices 34 qui occupent chacun sensiblement le tiers de la section du corps 3, entre trois ailettes qui rendent la coupelle 33 solidaire de ce corps. Le liquide contenu dans le soufflet 12 est alors libre d'atteindre une face amont 62 du tampon 6, par l'intermédiaire de la chambre de répartition 31, pour peu que l'utilisateur crée une surpression dans le flacon en manipulant le soufflet 12. Dans cette position l'étanchéité reste assurée sur le pourtour du corps 3 par le fait que les deux godrons 32 supérieurs s'enclipsent dans les rainures du col 5, tandis que le troisième (inférieur cette fois) vient buter contre un épaulement interne 53 de celui-ci.

Le déplacement de la tête de distribution entre ses deux positions longitudinales différentes à l'intérieur du col 5 s'obtient, de la position de sécurité à la position de disponibilité, par action sur le bouchon 7 coiffant l'ensemble, lors de la première utilisation.

La configuration du bouchon 7 ressort des figures 1 et 3. Du côté interne, en moyens coopérant avec l'embout 4 en toutes circonstances, il comporte un pion central 71 qui s'engage légèrement dans l'extrémité évasée du canal 41, une couronne annulaire 72 qui le guide en centrage sur la face externe d'un prolongement conique 43 de la base 42 de l'embout 4, traversé axialement par le canal 41, et une autre couronne annulaire 73, de plus large diamètre, qui vient prendre appui sur la face extérieure de la base 42 en sa face plane supérieure.

Le bouchon 7 est amovible par dévissage. Toutefois, il ne se visse pas directement dans le col 5 du flacon, mais dans la coque 8 mentionnée ci-dessus. Cette coque est rigide en toutes ses parties. En plus d'une partie principale 81, qui forme enveloppe autour du soufflet 12, elle comporte, en prolongement, un goulot 82 qui se monte fixe sur le col 5 du flacon. On peut voir sur la figure 1 qu'à cet effet, le goulot 82 et le col 5 présentent des surfaces de profils complémentaires, avec des épaulements ou godrons permettant un engagement ferme par clipsage élastique, sans qu'il soit là besoin d'assurer l'étanchéité.

Sur sa face périphérique extérieure, le col 82 de la coque 8 forme un filetage hélicoïdal 84 avec lequel coopère un filetage correspondant en surface interne du bouchon 7. Ce dernier comporte, d'origine, une bague d'inviolabilité 74 qui l'empêche d'être vissé au-delà de la position de sécurité illustrée par la figure 3, où la coupelle 33 est en contact étanche avec la paroi interne correspondante du flacon (siège 52) pour fermer le volume interne compressible du flacon.

Quand l'utilisateur ôte la bague d'inviolabilité 74, il est alors à même de visser le bouchon 7 jusqu'à la position d'utilisation, dans laquelle il vient en butée sur un épaulement supérieur 83 de la coque 81. Au cours de son déplacement longitudinal, le bouchon 7 entraîne avec lui la tête de distribution 2, et l'enfonce ainsi dans le flacon jusqu'à ouvrir les orifices 34. Par la suite, le bouchon peut être dévissé et vissé à chaque étape de distribution du produit, sans déplacer pour autant la tête de distribution. On peut remarquer ici que la coque 8 et le bouchon 7 présentent avantageusement le même diamètre extérieur, et ce non seulement pour améliorer l'esthétique, mais également pour faciliter la fabrication automatisée.

Revenant maintenant à la construction de la tête de distribution 2, on peut observer de la figure 1 que le tampon microporeux 6 s'engage dans le corps 3 de la tête 2, lors de l'assemblage quand elle n'est pas encore munie de l'embout 4, à partir de l'ouverture supérieure de son conduit cylindrique interne, qui est à surface lisse, et ce jusqu'à buter, le cas échéant, sur un épaulement interne 35.

A son extrémité supérieure (dans la position des figures 1 et 2), le corps 3 forme une collerette extérieure 36, qui est conçue pour venir en butée sur une extrémité supérieure tant du col 5 du flacon que du col 82 de la coque 8, quand la tête de distribution passe de la position de sécurité à la position de disponibilité, lors de la première utilisation. C'est sur cette collerette 36 que se réalise l'assemblage de l'embout 4, avantageusement par simple collage. Par ailleurs, mais du côté interne, la même collerette est conformée pour intégrer une structure 38, qui est moulée d'une seule pièce avec elle dans la matière du corps de nacelle 3, et dont l'une des fonctions est de retenir le tampon 6 par sa face aval 63.

Mais également, cette structure 38, de type très largement ajouré en étoile, a pour rôle de former entretoise entre le tampon 6 et la base 42 de l'embout 4. Elle réserve ainsi l'espace de la chambre de répartition 31, qu'elle limite par des parois évasées entre ses nervures radiales rejoignant la partie centrale cylindrique.

En outre, par sa périphérie et par la tranche de ses ailettes, elle sert à fixer et soutenir la membrane anti-bactérienne 9. On peut dire qu'elle constitue ainsi l'un des plateaux support de la membrane, en plaquant celle-ci contre la face inférieure de la base 42 de l'embout 4, qui elle forme l'autre plateau. Dans cette base, donc de l'autre côté de la membrane, il est ménagé des rainures annulaires 44 qui évitent un contact continu avec la membrane 9. De la sorte, on assure que la membrane, encadrée plane entre ses deux plateaux support, ne reste pas mouillée par la solution entre deux distributions successives, malgré l'effet de sa tension superficielle.

En ce qui concerne la conception de la coque 8, les figures illustrent deux modes de réalisation différents qui, cependant, sont identiques dans la coopération avec les autres organes du dispositif tels qu'ils sont illustrés par la figure 1.

Le premier de ces modes de réalisation est illustré par la figure 2a, dans une position de distribution, tête en bas, le bouchon 7 ayant été ôté par l'utilisateur (figure 2b). Dans ce cas, la coque 8 entoure le soufflet 12 sur l'ensemble de la longueur qu'il présente avant toute utilisation. D'autre part, elle est dépourvue de fond et elle présente une fente longitudinale 85.

Pour procéder à une distribution de gouttes, l'utilisateur glisse un doigt par cette fente pour atteindre le fond 13 du soufflet, à travers le fond ouvert 81 de la coque 8. Au fur et à mesure que le flacon se vide, il dispose toujours d'un accès suffisant par la fente 85 pour effectuer la compression longitudinale du soufflet, jusqu'à ce qu'il ne reste plus qu'une quantité correspond au volume mort inévitable.

La seconde variante a l'avantage de mieux exploiter les possibilités apportées par le tampon microporeux 6. L'accès au fond rigide 13 du soufflet s'effectue toujours par un fond ouvert de la coque 8. Mais celle-ci ne comporte plus de fente longitudinale. Elle est donc de pourtour cylindrique continu, ce qui entraîne une plus grande facilité de fabrication par le fait que, dans une chaîne industrielle, on peut aisément coller des étiquettes dessus. La coque 8 est alors associée à une cape de fermeture 87 s'adaptant pour fermer le fond de la coque autour du soufflet, mais ce, bien entendu, de manière amovible.

Il va de soi que la coque 8, exclusion faite de la cape de fermeture 87 éventuelle, est avantageusement réalisée d'une seule pièce, par moulage de matière plastique, suivant les technologies d'injection connues. Il en est de même pour le flacon 1, dès lors que l'on prévoit, dans la même matière, des épaisseurs de paroi différentes pour assurer d'une part la rigidité du col 5 et du fond 13, d'autre part une souplesse suffisante pour le reste pour autoriser la formation du soufflet flexible 12 par une opération de post-formage.

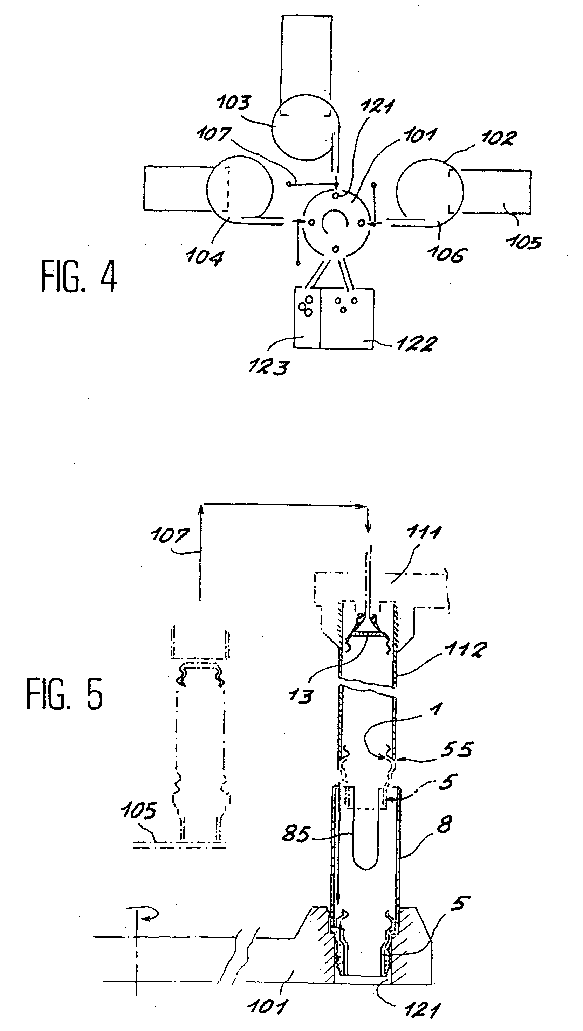

Toutefois, le flacon 1 présente une particularité inhabituelle. Il s'agit en l'occurrence d'un redent extérieur 55, qui à la base du col 5, forme une couronne encore rigide, donc une bague de renfort, dont le pourtour extérieur est en débord par rapport à l'encombrement du soufflet 12 et forme là une surface plane radiale. Son utilité est liée au procédé d'assemblage des différents éléments du dispositif de l'invention, comme il sera maintenant succinctement décrit en référence aux figures 4 et 5, sans détailler les organes de l'installation qui sont classiques en eux-mêmes pour des installations industrielles automatisées, dans leurs construction, relations et fonctions.

Conformément à ces figures, l'installation comporte un poste d'assemblage central en carrousel, dans lequel un plateau horizontal 101 est monté rotatif de manière à présenter à tour de rôle quatre alvéoles identiques de sa périphérie devant quatre postes disposés autour. On voit sur la figure 4 que ces derniers comprennent trois postes d'alimentation 102, 104, 104, chacun constitué, de manière en soit connue, par un bol vibrant qui reçoit les éléments à assembler d'une trémie 105 pour les faire passer un à un dans une goulotte les présentant correctement disposés pour être pris par un système de transfert évoqué par la référence 107. Au niveau du quatrième poste, l'assemblage du dispositif dans l'alvéole qui y parvient est terminé ; il est alors transféré et recueilli dans un bac des pièces bonnes 122, sauf pour les pièces détectées mauvaises, qui sont évacuées dans un bac de rebut 123.

Chacun des postes d'alimentation amène au poste d'assemblage, successivement au cours de la fabrication d'un dispositif suivant l'invention déterminé, d'abord la coque 8, que l'on voit sur la figure 5 calée dans l'alvéole 121, (la coque 8 étant ici du type à fente 85), puis le flacon 1, et enfin la cape de fond 87 complétant la coque de la figure 3. En variante, on peut trouver à ce troisième poste d'alimentation ou à d'autres postes qui seraient ajoutés au plateau tournant, des éléments à assembler tels que le bouchon 7 (qui serait alors présenté de l'autre côté du plateau tournant), l'embout 4, ou des enveloppes d'emballage.

La figure 5 montre l'installation au niveau du deuxième poste d'alimentation, celui qui correspond au traitement du flacon dans la chaîne d'assemblage, afin d'illustrer l'utilité de sa bague de renfort 55.

On peut y voir notamment que le flacon 1 y est manipulé tête en bas, alors que son soufflet se trouve protégé par un tube 112 qui fait partie de l'équipement du système de transfert. Par contre, par souci de rendre lisible une représentation qui est très schématique, on n'a pas fait apparaître qu'à ce stade, le flacon est déjà rempli du liquide à commercialiser et muni de la nacelle de montage du tampon 6, c'est-à-dire du corps 3 de la tête de distribution, non encore complété par l'embout 4, qui viendra se coller par-dessus ultérieurement.

On tire ici profit de la conception décrite pour le dispositif de l'invention. Avec son tampon microporeux, le corps 3 constitue un obturateur efficace pour le flacon tant qu'il n'est pas pressé sur le fond rigide 13 pour diminuer son volume interne et créer ainsi une surpression par rapport à l'extérieur. L'étanchéité est assurée par le tampon seul, dans le col rigide prolongeant le flacon, grâce à la compressibilité élastique du tampon sur son pourtour. Ceci donc, en combinaison avec la bague de renforcement externe 55 du col permet de le manipuler la tête en bas dans la machine d'assemblage. Cela permet également de maîtriser sa position dans la tête de distribution jusqu'à la position de sécurité sans aller jusqu'à la position d'utilisation.

En effet, le flacon 1 est transféré dans cette position, alors qu'il est suspendu par effet de ventouse sur son fond rigide, jusqu'à venir se placer au-dessus de la coque 8 déjà en place dans l'alvéole 121. Pendant tout le temps de son traitement dans ce poste, son soufflet est indéformable par le fait que l'extrémité inférieure du tube 112 vient buter en appui sur la baque 55, plus exactement sur sa face arrière en débord du soufflet. C'est aussi par un effet mécanique de pression exercé sur cette bague, par l'intermédiaire du tube 112 engagé avec jeu dans la coque 8, que l'on pousse le flacon pour forcer son col rigide 5 dans l'embouchure 82 de la coque 8, et ce sans toucher à la nacelle 3 qui est en position de sécurité.

La même bague de renfort 55 peut servir, en sens inverse, pour tenir le flacon par un outil de préhension approprié quand on enfonce la nacelle dans son col jusqu'à cette position de sécurité.

La description ci-dessus n'a été fournie qu'à titre d'exemple illustratif et nullement limitatif ; il est clair que l'on peut y apporter des modifications ou variantes sans pour autant sortir du cadre de la présente invention.

En particulier, on n'a pas mentionné ci-dessus que la coupelle 33, moulée d'une seule pièce dans la matière du corps de nacelle 3, est creusée vers l'axe du dispositif, encore que cela ressorte clairement des figures. Cette particularité améliore le fonctionnement en circulation du liquide vers le tampon 6.

D'autre part, au même niveau, les trois ailettes qui portent cette coupelle (entre les orifices 34 de passage du liquide) sont susceptibles de recevoir, sur leur tranche supérieure (figure 1), donc juste en aval du tampon 6, un filtre sélectif vis-à-vis d'un constituant du liquide qui ne devrait pas rester dans les gouttes expulsées. Ce serait le cas, par exemple pour une solution ophtalmique contenant un conservateur qu'il s'agirait de retenir par une membrane filtrante ou épuratrice par adsorption. Il est à noter toutefois que généralement, la présence du tampon 6 suffit à assurer l'effet nécessaire, en fonction de sa matière et ses dimensions de pores. On peut en plus envisager de lui faire subir un traitement adapté, en appliquant des technologies connues en elles-mêmes, en particulier quand le tampon est constitué en un feutre de fibres non tissées sous une densité correspondant à du polyéthylène à basse densité.

Enfin, on peut observer en se référant à la figure 1 que la base 42 de l'embout 4 présente un rebord qui limite une cuvette dans laquelle se loge la membrane 9. Cette dernière est ainsi à l'abri de détériorations d'origine mécanique jusqu'à la mise en place de l'embout sur le corps 3, lequel présente en périphérie de la structure 38 une forme complémentaire à celle de ce rebord.

## Revendications

1. Dispositif de conditionnement pour liquide à distribuer goutte à goutte, comportant un flacon (1) à paroi flexible, déformable manuellement en diminution progressive de son volume interne vers un col rigide (5) dans lequel se monte de manière étanche une tête de distribution (2) terminée par un canal (41) d'expulsion du liquide hors du flacon, **caractérisé en ce qu'**il comporte un tampon microporeux (6) s'insérant dans un corps (3) de ladite tête en travers de la section de passage du liquide entre ledit flacon (1) et ledit canal d'expulsion (41), en amont d'une chambre (31) de répartition du liquide, de section étendue de manière à recevoir au moins la dose d'une goutte de liquide et ménagée dans ledit corps (3) entre une surface aval (63) dudit tampon (6) et ledit canal d'expulsion (41), ledit tampon (6) étant réalisé en une matière inerte vis-à-vis du liquide et étant apte à créer une perte de charge régulatrice du flux de liquide poussé à travers lui par la pression engendrée dans le flacon (1) lors de chaque action en réduction de son volume et ladite chambre de répartition (31) étant d'un volume suffisant pour recevoir le liquide ayant ainsi traversé ledit tampon (6) et **en ce que** le flacon est isolé de toute possibilité de rentrée d'air en remplacement du liquide expulsé.

2. Dispositif selon la revendication 1, **caractérisé en ce que** ledit flacon (1) est limité par une paroi d'encombrement général cylindrique en forme de soufflet (12) déformable lonqitudinalement, terminé par un fond transversal rigide (13).

3. Dispositif selon la revendication 2, **caractérisé en ce que** ledit soufflet (12) est réalisé d'une seule pièce de matière moulable avec ledit col rigide (5) de montage de ladite tête de distribution (2) et avec une bague extérieure de renforcement (55) venant en débordement radial de l'encombrement général du soufflet (12) de manière à permettre sa préhension par une machine d'assemblage automatique sans toucher à la paroi plus fragile dudit soufflet.

4. Dispositif suivant l'une quelconque des revendications 1 à 3, **caractérisé en ce qu'**il comporte un élément filtrant aval, constitué par une membrane anti-bactérienne (9) qui est interposée intermédiaire entre ledit tampon (6) et ledit canal (41) en travers de ladite chambre (31), ladite membrane étant efficace pour isoler le flacon de toute possibilité de rentrée d'air en remplacement du liquide expulsé quand elle est imprégnée par ledit liquide.

5. Dispositif suivant l'une quelconque des revendications 1 à 4, **caractérisé en ce que** le corps (3) de ladite tête de distribution (2) est monté mobile entre deux positions longitudinales différentes à l'intérieur dudit col (5), comprenant une position de sécurité dans laquelle la communication entre le flacon (1) et ledit canal (41) par l'intermédiaire dudit tampon (6) est fermée étanche et une position de disponibilité où ladite communication est libre vers une face amont (62) dudit tampon (6) par l'intermédiaire de ladite chambre de répartition (31).

6. Dispositif suivant l'une quelconque des revendications 1 à 5, **caractérisé en ce que** ledit corps (3) de ladite tête de distribution (2) se montant étanche dans ledit col rigide (5) présente une forme tubulaire cylindrique dont toute la section transversale interne est occupée par ledit tampon sur un à deux tiers de sa longueur, le reste étant réservé à ladite chambre de répartition (31).

7. Dispositif suivant l'une quelconque des revendications 1 à 6, **caractérisé en ce que** ledit tampon (6) est constitué en une matière à base de résine organique présentant une légère souplesse permettant de l'engager en force dans ledit corps (3) de la tête de distribution et de le rendre ainsi étanche sur celle-ci.

8. Dispositif suivant la revendication 7, **caractérisé en ce que** ladite matière du tampon (6) est une mousse à haute porosité en pores ouverts ou un feutre équivalent, ménageant des microcanaux offerts au passage du liquide d'un diamètre moyen de pores compris entre 0,3 et 10 microns.

9. Dispositif suivant l'une quelconque des revendications 1 à 8, comportant un élément filtrant aval tel qu'une membrane anti-bactérienne suivant la revendication 4, **caractérisé en ce que** ladite membrane est soutenue plane entre deux plateaux ajourés formés respectivement par ledit corps (3) de ladite tête de distribution en aval de ladite chambre de répartition (31) et par un embout (4) qui en est solidaire à travers lequel est ménagé ledit canal d'expulsion (41).

10. Dispositif suivant la revendication 2, préférentiellement combinée à l'une quelconque des revendications 3 à 9, **caractérisé en ce que** ledit flacon (1) est monté par ledit col (5) à l'intérieur d'une coque extérieure de protection (8) l'enveloppant sur une majeure partie de sa longueur.

11. Dispositif suivant la revendication 10, **caractérisé en ce que** ladite coque (8) est de pourtour cylindrique continu et associée à une cape de fermeture (82) s'adaptant sur elle dans son prolongement, qui est amovible pour donner accès au fond (13) dudit flacon (1).

12. Dispositif suivant la revendication 10, **caractérisé en ce que** ladite coque (8) s'étend sur toute la longueur du flacon (1) et **en ce qu'**elle présente une fente longitudinale (85) livrant accès à la compression longitudinale du soufflet (12) à partir de son fond ouvert (81).

## Patentansprüche

1. Verpackungsvorrichtung für eine tropfenweise zu verteilende Flüssigkeit,
mit einem Fläschchen (1) mit flexibler Wand, das manuell in fortschreitender Reduzierung seines inneren Fassungsvermögens gegen einen festen Flaschenhals (5) verschiebbar ist,
in welchem in abdichtender Weise ein Verteilerkopf (2) montiert ist, an dessen Ende sich ein Kanal (41) zum Ausstoß der Flüssigkeit aus dem Fläschchen befindet,
**dadurch gekennzeichnet,**
- **dass** sie einen mikroporösen Stopfen (6) umfasst, der quer über den Durchflussabschnitt der Flüssigkeit zwischen dem Fläschchen (1) und dem Ausstoßkanal (41) in einen Körper (3) des Kopfes eingefügt ist und zwar zuströmseitig einer Kammer (31) zur Flüssigkeitsverteilung,
• die einen Abschnitt aufweist, der so erweitert ist, dass wenigstens die Dosis eines Flüssigkeitstropfens aufnehmbar ist, und
• die im Körper (3) zwischen einer abströmseitigen Oberfläche (63) des Stopfens (6) und dem Ausstoßkanal (41) angeordnet ist;
- wobei der Stopfen (6) aus einem der Flüssigkeit gegenüber inerten Material besteht und einen regulierten Druckabfall im Flüssigkeitsstrom, der über ihn durch den mit jeder Betätigung zur Reduzierung des Fassungsvermögens in dem Fläschchen (1) erzeugten Druck gefördert wird, bewirkt,
- und wobei die Verteilerkammer (31) ein ausreichendes Fassungsvermögen zur Aufnahme der den Stopfen (6) so durchlaufenden Flüssigkeit hat,
- und wobei das Fläschchen gegen jede Möglichkeit eines Luftrückstromes im Austausch gegen die ausgestoßene Flüssigkeit isoliert ist.

2. Vorrichtung nach Anspruch 1, **dadurch gekennzeichnet, dass** das Fläschchen (1) durch eine Wand mit zylinderförmigen Abmessungen in Form eines in Längsrichtung verformbaren Blasebalgs (12) abgeschlossen ist, die durch einen starren Querboden (13) abgeschlossen ist.

3. Vorrichtung nach Anspruch 2, **dadurch gekennzeichnet, dass** der Blasebalg (12) aus einem einzigen Stück formbaren Materials mit dem starren Flaschenhals (5) zur Montage des Verteilerkopfes (2)
und mit einem äußeren Verstärkungsring (55) gebildet ist, der sich radial über die Abmessungen des Blasebalgs (12) erstreckt und der durch eine automatische Montagemaschine greifbar ist, ohne die zerbrechlichere Wand des Blasebalgs zu berühren.

4. Vorrichtung nach irgendeinem der Ansprüche 1 bis 3, **dadurch gekennzeichnet, dass** sie ein zuströmseitiges Filterelement enthält, das aus einer antibakteriellen Membran (9) gebildet ist, die sich quer durch die Kammer (31) erstreckt und zwischen dem Stopfen (6) und dem Kanal (41) angeordnet ist, wobei die Membran das Fläschchen wirksam gegen jeden möglichen Lufteintritt im Austausch gegen die ausgestoßene Flüssigkeit abdichtet, wenn sie mit der Flüssigkeit voll gesogen ist.

5. Vorrichtung nach einem der Ansprüche 1 bis 4, **dadurch gekennzeichnet, dass** der Körper (3) des Verteilerkopfes (2) beweglich zwischen zwei verschiedenen Positionen in Längsrichtung im Inneren des Flaschenhalses (5) montiert ist, umfassend eine Sicherheitsposition, in der die Verbindung zwischen dem Fläschchen (1) und dem Kanal (41) durch den Stopfen (6) dichtend verschlossen ist, sowie eine Verfügbarkeitsposition, in der die Verbindung gegenüber einer zuströmseitigen Fläche (62) des Stopfens (6) durch die Verteilerkammer frei ist (31).

6. Vorrichtung nach einem der Ansprüche 1 bis 5, **dadurch gekennzeichnet, dass** der Körper (3) des Verteilerkopfes (2), der dicht in den genannten festen Flaschenhals (5) hineinmontiert ist, die Form eines Zylinderrohrs aufweist, dessen gesamter innen liegender Querschnitt auf ein bis zwei Drittel seiner Länge von dem Stopfen besetzt ist, wobei der Rest der Verteilerkammer (31) vorbehalten ist.

7. Vorrichtung nach einem der Ansprüche 1 bis 6, **dadurch gekennzeichnet, dass** der Stopfen (6) aus einem Material auf Basis eines organischen Harzes gebildet ist, das eine leichte Biegsamkeit aufweist, die es erlaubt, ihn unter Krafteinwirkung in den Körper (3) des Verteilerkopfes einzusetzen und ihn somit abdichtend auf diesem zu belassen.

8. Vorrichtung nach einem der Ansprüche 1 bis 7, **dadurch gekennzeichnet, dass** das Material des Stopfens (6) ein stark poröser, offenporiger Schaum oder ein äquivalenter Filzstoff ist, der Mikrokanäle für das Durchlaufen der Flüssigkeit mit einem mittleren Porendurchmesser von 0,3 bis 10 µm bereitstellt.

9. Vorrichtung nach einem der Ansprüche 1 bis 8, umfassend ein abströmseitiges Filterelement, insbesondere eine antibakterielle Membran nach Anspruch 4, **dadurch gekennzeichnet, dass** die Membran flach zwischen zwei lochgestanzten Tellern abgestützt ist, die jeweils gebildet sind von dem Körper (3) des Verteilerkopfes abströmseitig der Verteilerkammer (31) sowie von einem damit verbundenen Ansatzstück (4), durch welches der Ausstoßkanal (41) geführt ist.

10. Vorrichtung nach Anspruch 2, vorzugsweise mit wenigstens einem der Ansprüche 3 bis 9 kombiniert, **dadurch gekennzeichnet, dass** das Fläschchen (1) über den Flaschenhals (5) an die Innenseite einer äußeren Schutzschale (8) montiert ist, die es auf einem überwiegenden Teil seiner Länge umhüllt.

11. Vorrichtung nach Anspruch 10, **dadurch gekennzeichnet, dass** die Schale (8) einen durchgehend zylindrischen äußeren Umfang hat und mit einer sich in ihrer Verlängerung an sie anpassenden Verschlusshülle (82) verbunden ist, die zwecks Zugangs zum unteren Boden (13) des Fläschchens (1) abnehmbar ist.

12. Vorrichtung nach Anspruch 10, **dadurch gekennzeichnet, dass** sich die Schale (8) über die gesamte Länge des Fläschchens (1) erstreckt und dass sie eine längliche Spalte (85) aufweist, die Zugang zur Längskompression des Blasebalgs (12) von ihrem offenen Boden (81) aus gewährt.

## Claims

1. Device for packaging a liquid that is to be dispensed drop by drop, comprising a flexible-walled flask (1) that can be deformed manually to gradually decrease its internal volume towards a rigid neck (5) in which is leaktightly mounted a dispensing head (2) ending in a conduit (41) for expelling the liquid out of the flask, **characterized in that** this device comprises a microporous pad (6) inserted in a body (3) of the said head across the cross-sectional area for the passage of the liquid between the said flask (1) and the said expulsion conduit (41) upstream of a liquid-distribution chamber (31) of large cross-sectional area so as to accommodate at least the dose of one drop of liquid and formed in the said body (3) between a downstream surface (63) of the said pad (6) and the said expulsion conduit (41), the said pad (6) being made of a material that is inert with respect to the liquid and capable of creating a head loss which regulates the flow of liquid driven through it by the pressure generated in the flask (1) upon each action to reduce its volume and the said distribution chamber (31) having a large enough volume to accommodate the liquid which has passed through the said pad (6) and **in that** the flask is isolated from any possibility of air entering to replace the expelled liquid.

2. Device according to Claim 1, **characterized in that** the said flask (1) is bounded by a wall of cylindrical overall shape in the form of a longitudinally deformable bellows (12) ending in a rigid transverse bottom (13).

3. Device according to Claim 2, **characterized in that** the said bellows (12) is made as a single piece of material that can be moulded with the said rigid neck (5) for the mounting of the said dispensing head (2) and with an outer reinforcing ring (55) protruding radially from the overall size of the bellows (12) so as to allow it to be grasped by an automatic assembly machine without touching the more delicate wall of the said bellows.

4. Device according to any one of Claims 1 to 3, **characterized in that** it comprises a downstream filtering element consisting of an anti-bacterial membrane (9) which is inserted some way between the said pad (6) and the said conduit (41) across the said chamber (31), the said membrane being effective in isolating the flask from any possibility of air entering to replace the liquid expelled when this membrane is impregnated with the said liquid.

5. Device according to any one of Claims 1 to 4, **characterized in that** the body (3) of the said dispensing head (2) is mounted so that it can move between two different longitudinal positions inside the said neck (5), comprising a security position in which communication between the flask (1) and the said conduit (41) by way of the said pad (6) is closed off in a sealed manner and a service position in which there is freedom to communicate with an upstream face (62) of the said pad (6) by way of the said distribution chamber (31).

6. Device according to any one of Claims 1 to 5, **characterized in that** the said body (3) of the said dispensing head (2) mounted leaktightly in the said rigid neck (5) has a cylindrical tubular shape, the entire internal cross-sectional area of which is occupied by the said pad over one to two thirds of its length, the remainder being reserved for the said distribution chamber (31).

7. Device according to any one of Claims 1 to 6, **characterized in that** the said pad (6) is made of a material based on an organic resin with a slight amount of flexibility to allow it to be forcibly engaged in the said body (3) of the dispensing head and thus sealed against it.

8. Device according to Claim 7, **characterized in that** the said material of the pad (6) is a highly porous open-pore foam or an equivalent felt, forming microducts offered for the passage of the liquid with a mean pore diameter of between 0.3 and 10 microns.

9. Device according to any one of Claims 1 to 8, comprising a downstream filtering element such as an anti-bacterial membrane according to Claim 4, **characterized in that** the said membrane is supported flat between two perforated plates formed respectively by the said body (3) of the said dispensing head downstream of the said distribution chamber (31) and by a nozzle (4) secured thereto and through which the said expulsion conduit (41) is formed.

10. Device according to Claim 2, preferably combined with any one of Claims 3 to 9, **characterized in that** the said flask (1) is mounted via the said neck (5) inside a protective outer casing (8) which envelopes it over most of its length.

11. Device according to Claim 10, **characterized in that** the said casing (8) has a continuous cylindrical periphery and is associated with a closure cover (82) which fits onto it as a continuation thereof and is removable to give access to the bottom (13) of the said flask (1).

12. Device according to Claim 10, **characterized in that** the said casing (8) extends over the entire length of the flask (1) and **in that** it has a longitudinal slot (85) providing access for longitudinally compressing the bellows (12) from its open bottom (81).
